Europäisches Patentamt

European Patent Office

Office européen des brevets

(11) Veröffentlichungsnummer: **0 256 512 B1**

(12) **EUROPÄISCHE PATENTSCHRIFT**

(45) Veröffentlichungstag der Patentschrift: **31.03.93**

(21) Anmeldenummer: **87111694.3**

(22) Anmeldetag: **12.08.87**

Die Akte enthält technische Angaben, die nach dem Eingang der Anmeldung eingereicht wurden und die nicht in dieser Patentschrift enthalten sind.

(51) Int. Cl.⁵: **C12N 15/00**, C12P 1/00,
//C12N5/00,(C12P1/00,
C12R1:91)

(54) **Immortalisierung durch DNS-Übertragung.**

(30) Priorität: **12.08.86 DE 3627326**

(43) Veröffentlichungstag der Anmeldung:
**24.02.88 Patentblatt 88/08**

(45) Bekanntmachung des Hinweises auf die
Patenterteilung:
**31.03.93 Patentblatt 93/13**

(84) Benannte Vertragsstaaten:
**AT BE CH DE ES FR GB GR IT LI LU NL SE**

(56) Entgegenhaltungen:
**EP-B- 0 093 436**
**WO-A-86/03780**
**US-A- 4 652 522**

**BIOLOGICAL ABSTRACTS, Band 82, Zusammenfassung Nr. 108771; H. ABKEN et al.: "Immortalization of human lymphocytes by fusion with cytoplasts of transformed mouse L cells", & J. CELL. BIOL. 103 (3). 1986. 795-806**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Willecke, Klaus, Prof. Dr.rer.nat., Dipl.- Chem.**
**Taubenstrasse 47 a**
**W-4300 Essen 17(DE)**
Erfinder: **Abken, Hinrich Johann, Dr.med.**
**Ehrenaue 35**
**W-4300 Essen 1(DE)**
Erfinder: **Jungfer, Herbert, Dr. med.**
**Beringerweg 10**
**W-8132 Tutzing(DE)**
Erfinder: **Barchet, Heinrich**
**Bräuhaus Strasse 3**
**W-8132 Tutzing(DE)**

HYBRIDOMA, Band 3, Nr. 2, 1984, Seiten 107-118, Mary Ann Liebert, Inc.; Z.L. JONAK et al.: "Production of continuous mouse plasma cell lines by transfection with human leukemia DNA"

NATURE, Band 284, 3. April 1980, Seiten 418-421, Macmillan Journals Ltd; G.M. CO-OPER et al.: "Transforming activity of DNA of chemically transofrmed and normal cells"

PROC. NATL. SCI. USA, Band 85, Januar 1988, Seiten 468-472; H. ABKEN et al.: "Immortalization of human lymphocytes by transfection with DNA from mouse L929 cy-toplasts"

(74) Vertreter: **Huber, Bernhard, Dipl.-Chem. et al Patentanwälte H. Weickmann, Dr. K. Fincke F.A. Weickmann, B. Huber Dr. H. Liska, Dr. J. Prechtel Kopernikusstrasse 9 Postfach 86 08 20 W-8000 München 86 (DE)**

EP 0 256 512 B1

**Beschreibung**

Die Erfindung betrifft ein Verfahren zur Herstellung von immortalisierten, das heißt permanent züchtbaren Zell-Linien.

Normale menschliche und tierische Zellen haben eine begrenzte Lebenszeit in Kultur. Sie können sich mit einer bestimmten Verdopplungszeit vermehren oder als ruhende Zellen in der G0/G1-Phase des Zell-Zyklus verbleiben. Bei sich vermehrenden Zellen ist der Beginn der Alterung in Kultur vom Zelltyp und vom Alter, sowie der Spezies des Spenders abhängig. Tumorbildende Zell-Linien können unbegrenzt kultiviert werden, unterscheiden sich aber in zahlreichen Eigenschaften von normalen, alternden Zellen.

Beispielsweise befinden sich menschliche Lymphozyten aus dem peripheren Blut in der Ruhephase (G0/G1) des Zell-Zyklus, können aber in Kultur auf verschiedene Weise zum Eintritt in die Vermehrungsphase (S) und zur Reifung (Differenzierung) angeregt werden. B-Lymphozyten in der Ruhephase können durch Antigene, B-Lymphozytenwachtumsfaktoren oder durch T-Lymphozyten vermittelte Stimulation mit Hilfe eines Wachstumsfaktors (Interleukin) zur Vermehrung und Reifung veranlaßt werden. Ebenso können T-Lymphozyten durch Zellteilung anregende Substanzen (Mitogene) oder spezifische Wachstumsfaktoren zur Vermehrung und Reifung in Kultur angeregt werden. Langzeitkulturen normaler Lymphozyten sind von der Anwesenheit entsprechender Wachstumsfaktoren oder Antigene im Kulturmedium abhängig. Ferner können menschliche Lymphozyten auch durch Infektion mit Viren in Kultur zur permanenten Vermehrung angeregt werden. Die Zellen verlieren dabei einige Eigenschaften normaler Lymphozyten und erhalten neue Eigenschaften, die sie mit tumorbildenden Lymphozyten gemeinsam haben. Das Epstein-Barr Virus vermag nach Infektion in Kultur eine Untergruppe menschlicher B-Lymphozyten, die den C3-Rezeptor und den Epstein-Barr Virus Rezeptor ausprägen, zur ständigen Vermehrung anzuregen. Dabei werden die Lymphozyten auf einer frühen Differenzierungsstufe angehalten. Das menschliche T-Zell Leukämie/Lymphom Virus (HTLV), Typ I, II, infiziert dagegen fast ausschließlich T-Lymphozyten, die durch eine bestimmte Oberflächenkomponente, dem OKT4 + Marker, gekennzeichnet sind. Einige Zell-Linien bleiben auch nach HTLV-Infektion für ihre Vermehrung von T-Lymphozyten-Wachstumsfaktoren abhängig.

Ferner lassen sich permanente Zell-Linien durch Zell/Zell-Fusionen gewinnen. Dabei werden z.B. Lymphozyten mit Zellen unbegrenzt teilungsfähiger Linien (z.B. Myelom-Zellen, Lymphom-Zellen, Epstein-Barr Virus infizierten lymphoiden Zellen) fusioniert und anschliessend so selektioniert, daß die entstandenen Hybrid-Zellen sich bevorzugt vermehren können. Die Hybridzellen haben den doppelten Chromosomensatz einer Ausgangszelle und verlieren z.T. in den folgenden Zellgenerationen die Chromosomen der normalen Ausgangszelle (Lymphozy-ten). Dabei können gewünschte Eigenschaften der HybridZellen, wie z.B. die Produktion von Antikörpern, Oberflächenproteinen, sezernierten Proteinen oder andere Sytheseleistungen der Zelle wieder verloren gehen. Diese Zellfusions-Verfahren zur Gewinnung von permanenten Zell-Linien lassen sich auch nur auf Lymphozyten und neuronale Zellen anwenden.

Aus Hybridoma 3(2), 1984, 107-117 läßt sich entnehmen, daß bei der Immortalisierung von Zellen DNA die essentielle Komponente darstellt. Es finden sich dort jedoch keine Hinweise darauf, daß eine DNA aus Cytoplasma oder Cytoplasten wesentlich besser zur Immortalisierung geeignet ist als Gesamt-DNA oder Kern-DNA, also die weit überwiegende Menge an DNA der Zelle.

Aus der EU-A1 83 104 243.7 ist es bereits bekannt, immortalisierte humane oder tierische Zellen aus normalen Zellen durch Fusion mit einem nicht vermehrungsfähigen Fragment einer transformierten Zelle und Züchtung in einem Medium ohne Selektionssubstanzen herzustellen. Als nicht vermehrungsfähige Fragmente können hierbei auch Cytoplasten verwendet werden. Hierdurch gelingt es, beliebige tierische und menschliche Zellen zu immortalisieren und gleichzeitig die Ausbeute durch Wegfall der Selektionssubstanzen zu steigern. Außerdem wird die oben erwähnte, bei der Zell-Zell-Fusion im Laufe der Weiterzüchtung häufig auftretende nachträgliche Veränderung der Zelleigenschaften vermieden. Jedoch wäre eine weitere Verbesserung der Ausbeute und der Permanenz immortalisierter Zellen erwünscht.

Der Erfindung liegt daher die Aufgabe zugrunde, die Immortalisierungsausbeute und die Eigenschaften der immortalisierten Zellen weiter zu verbessern.

Gelöst wird diese Aufgabe erfindungsgemäß durch ein Verfahren zur Immortalisierungvon humanen oder tierischen Zellen durch Transfektion mit einem nicht vermehrungsfähigen Fragment einer permanent züchtbaren Zelle und Züchtung derselben in einem Kulturmedium ohne Selektionssubstanzen, welches dadurch gekennzeichnet ist, daß man aus dem Cytoplasma einer permanent züchtbaren menschlichen oder tierischen Zelle gewonnene DNS in die zu immortalisierenden Zellen transfiziert.

Erfindungsgemäß erhält man unbegrenzt in Kultur vermehrbare Zell-Linien, wobei bestimmte Nachteile der Etablierung von Zell-Linien durch Zellfusion oder durch chemische Carcinogene sowie durch Infektion mit tranformierenden Viren vermieden werden. Die nach dem erfindungsgemäßen Verfahren erhaltenen Zell-Linien haben weitgehend ihre normalen Eigenschaften beibehalten, was eine vielfältige Verwendung der

3

Zellen ermöglicht.

Die erhaltenen Zell-Linien wachsen unabhängig von zellspezifischen, exogen zugegebenen Wachstumsfaktoren.

Das erfindungsgemäße Verfahren kann zur Immortalisierung beliebiger humaner oder tierischer Zellen verwendet werden, einschließlich von Zellen des Immunsystems wie Lymphozyten und dgl., Bindegewebszellen, differenzierte Organzellen, Epithelzellen usw.

Die Immortalisierung dieser Zellen erfolgt erfindungsgemäß durch Einführung (Transfektion) einer bestimmten Art von DNS, welche aus dem Cytoplasma von permanent züchtbaren menschlichen oder tierischen Zellen gewonnen wird. Es wird also nicht die Zellkern-DNS (genomische DNS) verwendet, sondern lediglich die aus dem den eigentlichen Kern umgebenden Cytoplasma isolierbare DNS. Vorteilhaft geht man deshalb zur Gewinnung dieser DNS von Cytoplasten aus. Als permanent züchtbare menschliche und tierische Zellen zur Gewinnung der transfizierten DNS können Tumorzellen oder auf andere Weise immortalisierte nicht tumorigene Zellen, beispielsweise nach dem erfindungsgemäßen Verfahren bereits hergestellte immortalisierte Zellen verwendet werden. Besonders gute Ergebnisse werden erhalten unter Verwendung von DNS aus Cytoplasten transformierter Maus L-Zellen (L-929 Zellen, ATCC CCL 1), Maus Myelom-Zellen (Ag8.653 Zellen ATCC CRL-1580), Ehrlich-Aszites-Zellen (EAZ, ATCC CCL 77) oder Mensch Carcinom-Zellen (z.B. Hela 229, ATCC CCL 2.1). Angesichts der außerordentlich großen Vielzahl von existierenden Tumorzell-Linien und immortalisierten nicht tumorigenen Zell-Linien ist eine erschöpfende Aufzählung nicht möglich. Die Eignung der cytoplasmatischen DNS der permanent züchtbaren Zell-Linie läßt sich jedoch auf einfache Weise durch Isolierung dieser DNS und Prüfung der Wachstumseigenschaften von damit transfizierten normalen Zellen feststellen.

Die Gewinnung der cytoplasmatischen DNS erfolgt ausgehend vom isolierten Cytoplasma oder vorzugsweise Cytoplasten. Geht man von Cytoplasten aus, so werden diese erst lysiert und das lösliche Lysat mit einer Proteinase inkubiert. Aus dem löslichen Anteil der Inkubationsmischung wird dann die DNS nach bekannten Methoden abgetrennt. Geeignete Methoden sind Isolierung über Dichtegradienten, z. B. mit CsCl (Nucleid Acid Research and Molekular Biology 3 (1964) 1 bis 32), Affinitätschromatographie oder elektrophoretische Auftrennung ("Molecular Cloning", ed. Maniatis, Fritsch, Sambrook, Cold Spring Harbour Lab, 1982) und Extraktion mit DNS-Lösungsmitteln. Bevorzugt erfolgt die Abtrennung, indem man mit einem DNS-Lösungsmittel extrahiert, den Extrakt mit RNase inkubiert und erneut freigesetzte DNS mit einem DNS-Lösungsmittel extrahiert. Durch Fällung, beispielsweise mit Alkohol, läßt sich die cytoplasmatische DNS dann isolieren. Geht man nicht von Cytoplasten, sondern von bereits getrennt isoliertem Cytoplasma aus, so entfällt natürlich die anfängliche Lyse des Vesikels.

In einer weiteren bevorzugten Ausführungsform wird die zu transfizierende DNS aus einer von nukleärer DNS freien Cytoplasmafraktion verwendet. Die Herstellung dieser Cytoplasmafraktion kann beispielsweise durch Lyse der Zellen mit Natriumdodecylsulfat (SDS) oder Ethylphenylpolyethylenglycol (NP40, Roth, BRD) in Gegenwart von 3 mM/l $MgCL_2$ (Meth.Enz. 31 (1974) 253-262) oder durch Lyse der Zellen mit NaCl und SDS (Hirt-Extraktion, J.Mol.Biol. 26 (1967) 365-369) und anschliessende Zentrifugation erfolgen. Der so gewonnene Überstand ist frei von nukleärer DNS. Aus diesem Überstand kann die zu transfizierende DNS, wie oben beschrieben, gewonnen werden.

In einer weiteren besonders bevorzugten Ausführungsform wird die zu transfizierende DNS aus einer mitochondrienfreien Fraktion des Cytoplasmas verwendet. Diese Fraktion kann beispielsweise aus Cytoplasten über Gradientenzentrifugation mit einem Sucrosegradienten isoliert werden.

Überraschenderweise zeigt sich, daß die immortalisierende Aktivität einer mitochondrienfreien Fraktion der DNS des Cytoplasmas um ca. den Faktor 10 höher ist als die Aktivität, die mit DNS aus Cytoplasten erhalten werden kann.

Als DNS-Lösungsmittel können die dem Fachmann bekannten, hierfür geeigneten Lösungsmittel verwendet werden, soweit sie sich zur Extraktion aus dem Medium, in dem die DNS enthalten ist, eignen. Für wäßrige Lösungen eignen sich als DNS-Lösungsmittel besonders Phenol oder/und Chloroform-Isoamylalkoholgemische zur Extraktion.

Die zu immortalisierende Zelle wird vorzugsweise in zum Wachstum bzw. zur Teilung angeregtem Zustand in die Transfektion mit der cytoplasmatischen DNS eingesetzt. Die Überführung in diesen Zustand erfolgt nach dem Fachmann hierfür bekannten Methoden, beispielsweise durch Zusatz von "poke-weed mitogen" zu dem die Zellen enthaltenden Medium. Als Proteinase wird eine der dem Fachmann bekannten Proteinasen, vorzugsweise Proteinase K, verwendet. Als RNase wird RNase A bevorzugt.

Verwendet man Lymphozyten als zu immortalisierende normale Zellen, so werden sie bevorzugt in der Proliferationsphase I eingesetzt. Als Proliferationsphase I wird die Phase bezeichnet, in der eine Poliferation nach Stimulierung von Lymphozyten mit Hilfe von Mitogenen (z. B. poke-weed mitogen), Antigenen usw. erreicht wird.

Besonders gute Ergebnisse ergaben sich, wenn man aus erfindungsgemäß erhaltenen immortalisierten Zellen (primäre Transfektanten) gewonnene Gesamt-DNS in einem weiteren Verfahrensschritt mit normalen humanen oder tierischen Zellen transfiziert und auf diese Weise sekundäre Transfektanten gewinnt, welche in besonders hohen Ausbeuten permanent weiterzüchtbar sind. Als normale Zellen werden für die zweite Transfektion entweder Zellen der gleichen Zellart wie für die erste Transfektion oder hiervon verschiedene Zellen eingesetzt.

Zur Gewinnung von Cytoplasten werden Zellen in Kultur durch Cytochalasin B (einem Wirkstoff aus Pilzen) im Medium veranlaßt, Vesikel (Cytoplasten) zu bilden, die sich auf der Zelloberfläche abschnüren. Unter Einwirkung von Zentrifugalkräften reißt die Verbindung zwischen Cytoplasten und verbliebener Zelle ab und die Cytoplasten lassen sich von den kernhaltigen Restzellen (Karyoplasten) durch Zentrifugation im Dichtegradienten isolieren. Diese Methoden der Cytoplastenisolierung sind z.B. beschrieben von Wigler, M.M. und Weinstein, I.B. in "A preparative Method für obtaining enucleated mammalian cells" (Biochem. Biophys. Res. Comm. 63: 669-674, 1975). Anschließend werden nach dem Verfahren der Erfindung die Cytoplasten lysiert, die DNS daraus isoliert und in die Ziel-Zelle übertragen. Alternativ kann erfindungsgemäß die verwendete DNS aus permanent züchtbaren Zellen gewonnen werden, die nach dem Verfahren der Erfindung erhalten wurden. Dabei wird die gesamte, in der Zelle enthaltene DNS nach dem Fachmann bekannten Methoden isoliert. Die DNS wird dann nach bekannten Verfahren in die Zielzelle übertragen.

Ein wesentliches Merkmal der Erfindung liegt darin, daß die nach DNS-Aufnahme sich permanent vermehrenden Zellen nicht mit Hilfe von Selektionssubstanzen in Kulturmedien selektioniert werden. Hierin unterscheidet sich das erfindungsgemäße Verfahren von der Zellfusions-Technik, bei der für die ausschließliche Vermehrung der Hybridzelle und gegen die Vermehrung der Ausgangszelle selektioniert werden muß. Es gibt zahlreiche permanent wachsende Zell-Linien, die unempfindlich gegenüber Selektionssubstanzen im Kulturmedium sind und damit nicht ohne weiteres für Zellfusionen mit normalen Zellen verwendet werden können. Nach dem erfindungsgemäßen Verfahren entfällt diese Beschränkung und es kann aus Cytoplasten dieser Zellen DNS isoliert werden, die für die Etablierung von permanent sich vermehrenden Zell-Linien eingesetzt werden kann.

Aus den Zellen der etablierten menschlichen und tierischen Linien können Zellprodukte in unbegrenzter Menge gewonnen werden, wie z.B. Immunglobuline, Gerinnungsfaktoren, Lymphokine, Wachstumsfaktoren, Hormone, Enzyme, Oberflächenproteine sowie Glyko- und Lipoproteine, Saccharide usw.. Für diagnostische und therapeutische Zwecke können menschliche monoklonale Antikörper aus permanent sich vermehrenden menschlichen B-Lymphozyten gewonnen werden. Lymphokine können von menschlichen T-Lymphozyten und Makrophagen produziert werden. Ferner können die permanent sich vermehrenden Zellen zur Herstellung heterologer Genprodukte benutzt werden. Hierzu wird die genetische Information für das heterologe Genprodukt in die erfindungsgemäß erhaltenen, permanent sich vermehrenden Zellen übertragen und dort ausgeprägt. So konnte gezeigt werden, daß die genetische Information für das Enzym Xanthin-guanin-phosphotransferase, die von Genom der E.coli Bakterien, nicht aber vom Genom der Säugerzellen kodiert wird, in permanent sich vermehrende Zellen eingebracht werden kann und dort ausgeprägt wird. Die erhaltene Zell-Linie synthetisiert das bakterielle Enzym und enthält damit eine neue Stoffwechselaktivität. Außerdem können die erfindungsgemäß erhaltenen, unbegrenzt züchtungsfähigen Zell-Linien im Rahmen der Mutagenitäts- und Toxizitätstestung, sowie für die pharmakologische Prüfung von Wirksubstanzen genutzt werden.

Gemäß der Erfindung könnten Zellen bestimmten Differenzierungsgrades immortalisiert und in großem Maßstab gezüchtet werden, die nur in relativ geringen Mengen isoliert werden können. Auf diese Weise gelingt es, die DNS sowie RNS dieser immortalisierten Zellen mit gewünschten Stoffwechseleigenschaften oder Fähigkeiten zur Synthese gewünschter Zellprodukte in einer zur Isolierung der genetischen Information für das gewünschte Zellprodukt ausreichenden Menge zu gewinnen. Bei dieser Anwendungsform der Erfindung kann nach geeigneter Manipulation die genetische Information in andere eukaryonte Zellen oder in Bakterien sowie Pilze mit Hilfe eines Vektors übertragen und dort unter den hierfür entwickelten Bedingungen zur Produktion des gewünschten Zellprodukts ausgeprägt werden. Das gewünschte Zellprodukt kann dann aus dem heterologen Wirt in beliebiger Menge gewonnen werden.

Die folgenden Beispiele erläutern die Erfindung.

**Beispiel 1**

Immortalisierung menschlicher Lymphozyten aus peripherem Blut.

Material:

Cytochalasin B (Sigma), Lymphoprep® (Nyegaard, Oslo), Iscove's modifiertes Dulbecco's Minimal Eagle's Medium (Iscove's DMEM) (Boehringer Mannheim), fötales Kälberserum (Sebio), DEAE-Dextran (Pharmacia), Ficoll (Pharmacia), Polyäthylenglycol (Boehringer Mannheim)

Methoden:

1. Isolierung menschlicher Lymphozyten aus peripherem Blut.

Peripheres Blut erwachsener Spender wurde in bekannter Weise heparinisiert und dreifach in 3 mM Zitronensäure, 100 mM Dextrose, 70 mM NaCl, 30 mM Nacitrat, pH 6,1, verdünnt. Die Lymphozyten wurden von den übrigen kernhaltigen Zellen (Granulozyten, Monozyten u.a.) durch Zentrifugation in einem Lymphoprep® (Metrizoat) Gradienten (Nyegaard, Oslo) bei 400 x g, 35 min getrennt (Boyum, A.:A one-stage procedure for isolation of granulocytes and lymphocytes from human blood. Scand. J. Clin. Invest. 21, suppl. 97: 51-76, 1968). Die Lymphozyten wurden dreimal in phosphate buffered saline (PBS) (136 mM NaCl, 2,7 mM KCl, 1,5 mM $KH_2PO_4$, 6,5 mM $Na_2HPO_4$, 0,4 mM $MgSO_4$, 0,7 mM $CaCl_2$, pH 7,4) gewaschen und in Iscove's DMEM, 4 mM L-Glutamin, 1 mM Pyruvat, 1 mM Oxalessigsäure, 0,1 U/ml Insulin, 10 $\mu$g/ml Transferrin, 15 % fötalem Kälberserum bei einer Zelldichte von 3 x $10^6$ Zellen/ml bei 37 C, 5 % $CO_2$ kultiviert.

2. Isolierung der immortalisierten DNS.

Die immortalisierende DNS wird aus Cytoplasten von Maus L929-Zellen gewonnen. Die Induktion und Isolierung von Cytoplasten transfizierter Zellen durch Inkubation mit Cytochalasin B ist in der Literatur beschrieben (Allikmets, E., Vasil'ev, I., Rovenskii, I.: Effekt of cytochalasins on the surface topography of neoplastic cells in suspension. Bull. Exp. Biol. Med. (Engl. Trans. Biull. Eksp. Biol. Med.) 95: 84 - 87, 1983; Wigler, M.M., Weinstein, I.B.: A preparative method für obtaining enucleated mammalian cells. Biochem. Biophys. Res. Comm.63: 669-674, 1975). Bevorzugt wird folgende Durchführung:
Exponentiell wachsende L929-Zellen werden trypsiniert, in serumfreiem DMEM gewaschen und in einer Dichte von $10^6$ Zellen/ml mit Cytochalasin B (50 $\mu$g/ml) bei 37°C inkubiert. Innerhalb von 90 sec bilden sich Cytoplasten, die sich an der Oberfläche der Zellen abschnüren. Die Cytoplasten werden von den Zellen durch kräftiges Suspendieren getrennt. Die verbleibenden Zellen werden durch Zentrifugation bei 100 x g, 10 min sedimentiert. Die Cytoplasten im Überstand werden durch Zentrifugation bei 1.200 x g, 15 min sedimentiert und in serumfreiem DMEM, 15 % Ficoll (Pharmacia) resuspendiert. Von den verbliebenen kerhaltigen Vesikeln werden die Cytoplasten in einem Gradienten mit 2 ml 25% Ficoll, 2 ml 17% Ficoll, 0,5 ml 16% Ficoll, 0,5 ml 15% Ficoll, 2 ml 12,5% Ficoll in serumfreiem DMEM (von unten nach oben) durch Zentrifugation bei 100.000 x g, 60 min, 30°C getrennt. Die Cytoplasten-Bande in der 15 bis 16% Ficoll-Region wird gesammelt und zweimal in serumfreiem DMEM bei 1.200 x g für 15 min zentrifugiert.
Zur Isolierung der DNS wird das Cytoplastensediment in 200 $\mu$l serumfreiem DMEM resuspendiert und 5 ml 50 mM Tris, pH 7,2, 10 mM EDTA, 1,5 mM $MgCl_2$ hinzugegeben. Die Cytoplasten werden durch Zugabe von 2 $\mu$l NP40 (Roth) lysiert und das Lysat bei 14.000 x g, 15 min zentrifugiert. Der Überstand wird mit Proteinase K (100 $\mu$g/ml, Boehringer Mannheim) versetzt, 2 h bei 37°C inkubiert und mit Phenol zweimal extrahiert. Anschließend wird der Überstand mit RNase A (100 $\mu$g/ml, Boehringer Mannheim) 1 h bei 37°C inkubiert, Proteinase K (100 $\mu$g/ml, Boehringer Mannheim) hinzugegeben und nochmals 2 h inkubiert. Die DNS wird jeweils zweimal mit Phenol und Chloroform/Isoamylalkohol (24:1) und einmal mit Äther extrahiert, in Äthanol gefällt und in 10 mM Tris, 0,1 mM EDTA, pH 7,5, aufgenommen. Die Ausbeute beträgt ca. 5 $\mu$g DNS/$10^8$ Cytoplasten.

3. Übertragung immortalisierender DNS in menschliche Lymphozyten.

Die gemäß Methode 1. präparierten Lymphozyten (2 x $10^8$ Zellen) werden unter dem Fachmann bekannten Bedingungen durch "pokeweed mitogen" (PWM, 10 $\mu$g/ml) zum Wachstum angeregt und zwei Tage später in zwei Parallelkulturen (A,B) zu je $10^8$ Zellen getrennt. Die Zellen der Kultur A werden mit

6

gemäß 2. erhaltener DNS aus Cytoplasten der L929-Zellen inkubiert. Die Zellen der Kultur B werden genauso behandelt wie die Kultur A, ohne daß eine zu übertragende DNS im Inkubationsmedium vorhanden ist. Anschließend werden die Lymphozyten dreimal in "HEPES buffered salt saline" (HeBS; 20 mM HEPES, 137 mM NaCl, 0,5 mM KCl, 3 mM Glukose, pH 7,1) gewaschen und mit DEAE-Dextran (MW 500.000, 0,3 mg/ml HeBS) bei einer Dichte von $10^6$ Zellen/ml 10 min bei 37°C inkubiert. Die Zellen werden zweimal in HeBS bei 37°C gewaschen. Die DNS aus L929-Cytoplasten (3 $\mu$g) wurde zu 100 $\mu$l 250 mM $CaCl_2$ gegeben und diese Lösung tropfenweise zu doppelt konzentriertem HeBS (100 $\mu$l) pipettiert. Die Lymphozyten werden in einer Dichte von 5 x $10^6$ Zellen/ml 45 min bei 37°C mit dieser DNS-Lösung inkubiert. Anschließend werden die Lymphozyten einmal in HeBS bei 37°C gewaschen, in 0,8 ml HeBS resuspendiert und 90 sec mit 0,5 ml Polyäthylenglycol (MW 4.000, 500 mg/ml HeBS, pH 7,1) bei 37°C inkubiert. Über einen Zeitraum von 5 min werden 5 ml warmes HeBS hinzupipettiert, die Lymphozyten zweimal in HeBS gewaschen, in einer Dichte von $10^7$ Zellen/ml in Iscove's DMEM, 4 mM L-Glutamin, 1 mM Pyruvat, 1 mM Oxalessigsäure, 0,1 U/ml Insulin, 10 $\mu$g/ml Transferrin, 15% fötales Kälberserum in 24-Loch Microtiterschalen (1 ml/Vertiefung) ausgesät und bei 37°C, 5 % $CO_2$ inkubiert. Während der nächsten zwei Wochen werden 100 $\mu$l Medium/Vertiefung alle 2 Tage durch frisches Medium ersetzt. In der 5. Woche werden die Kulturen dreifach verdünnt. Die anschließend auswachsenden immortalisierten Zell-Linien werden weiterhin in Iscove's DMEM, in RPMI 1640 Medium, je 15% fötalem Kälberserum, oder in serumfreien Medien (HB-104, NEN-DuPont; DMEM-SFM-2, Boehringer Mannheim) kultiviert.

Ergebnis:

In der Kultur A wurden 3 Tage nach DNS-Übertragung Lymphozytenkolonien beobachtet, die in den folgenden 5 Tagen an Größe und an Zell-Zahl (bis zu 500 Zellen/Kolonie) zunahmen. Diese Kolonien zerfielen in mehrere kleinere Kolonien, wovon einige etwa ab 20 Tagen nach DNS-Übertragung kontinuierlich zu wachsen begannen. Am Tag 30 nach DNS-Übertragung waren in allen Vertiefungen der Microtiterplatte mehrere Kolonien lymphoider Zellen erkennbar (Tabelle 1). Die Zellen konnten 5 Wochen nach DNS-Übertragung dreifach verdünnt werden. In der folgenden Zeit wurden die Zellen bis zu 7 Monaten in Kultur vermehrt, ohne daß eine Alterung der Zellen zu beobachten war.

In Kultur B wurden nach Schein-DNS-Übertragung keine Vermehrung der Lymphozyten und keine kontinuierlich wachsende Kolonien lymphoider Zellen beobachtet. Die Zellen alterten innerhalb von 4 Tagen nach Schein-DNS-Übertragung und starben ab.

Tabelle 1

Zusammenfassung der Ergebnisse zur Immortalisierung menschlicher Lymphozyten des peripheren Bluts

|  | eingesetzte Lymphozyten | immortalisierte lymphoide Kolonien | eingesetzte DNS ($\mu$g) | immortalisierte Kolonien/$10^7$Zellen/ µg DNS |
|---|---|---|---|---|
| Kultur A | $10^8$ | 52 | 3 | 1,7 |
| Kultur B | $10^8$ | 0 | 0 | 0 |

**Beispiel 2**

Immortalisierung menschlicher B-Lymphozyten aus dem peripheren Blut

Material:

Sepharose 6MB, CNBr-aktiviert (Pharmacia), Maus monoklonaler Antikörper anti-human-pan-B-Lymphozyten (Miles), humanes Serumalbumin (Serva) Methoden:
Die Isolierung menschlicher Lymphozyten aus dem peripheren Blut erfolgte wie in Beispiel 1 beschrieben.

1. Isolierung menschlicher B-Lymphozyten.

Für die Gewinnung der B-Lymphozyten wurde die Lymphozyten-Präparation mit Hilfe der Affinitätschromatographie aufgetrennt. Dazu wird folgendes Verfahren angewendet.
Maus monoklonaler anti-human-pan-B-Lymphozyten Antikörper wird in 0,1 M $NaHCO_3$, 0,5 M NaCl, pH 8,5 gelöst und mit CNBr-aktivierter Sepharose 6MB (1 ml Antikörper/ml Gel) bei 4°C über Nacht inkubiert. Die Sepharose wird dreimal 30 min in 0,1 M $NaHCO_3$, 0,5 M NaCl, pH 8,5 und dreimal 30 min in 0,1 M Na-acetat, 0,5 M NaCl, pH 4,5 gewaschen. Um die noch freien reaktiven Gruppen zu blockieren wurde die Sepharose mit 0,2 M Glukosamin, 0,1 M Na-acetat, 0,5 M NaCl, pH 4,5, 2 Stunden inkubiert. Die Sepharose wird zweimal in "phosphate buffered saline" (PBS), 0,2% Humanserumalbumin (HSA) gewaschen. Zur Isolierung der B-Lymphozyten wurden die Zellen der Lymphozytenpräparation aus dem peripheren Blut in PBS, 0,2% HSA gewaschen, auf die Sepharose-Säule geschichtet und 15 min inkubiert. Die nicht an der Säule gebundenen Zellen (T-Lymphozyten u.a.) wurden mit 50 ml PBS, 0,2% HSA ausgewaschen. Um die gebundenen Zellen (B-Lymphozyten) zu gewinnen, wurde die Sepharose mit 2 ml PBS, 0,2% HSA, 10 mg/ml IgG bei 37°C 20 min inkubiert. Die B-Lymphozyten wurden mit PBS, 0,2% HSA, 10 mg/ml IgG ausgewaschen.
Die Reinheit der B-Lymphozytenpräparation wird nach dem Fachmann bekannten Methoden der indirekten Immunfluoreszenz bestimmt. In dieser Präparation trugen über 90 % der Zellen B-Lymphozyten-Marker.

2. Immortalisierung der B-Lymphozyten.

Die B-Lymphozyten werden durch Inkubation mit "pokeweed mitogen" (PWM, 10 $\mu$g/ml) zum Wachstum angeregt. Zwei Tage später wird die Kultur in zwei Parallelkulturen (A,B) zu je 3 x $10^7$ Zellen getrennt. In Zellen der Kultur A wurde DNS aus L929-Cytoplasten (1$\mu$g) übertragen (wie in Beispiel 1 beschrieben). Die Zellen der Kultur B wurden gleich behandelt, ohne daß DNS angeboten wurde. Die Zellen jeder Kultur wurden in einer Dichte von 5 x $10^6$ Zellen/ml in 24-Loch Microtiterplatten (1 ml/Vertiefung) ausgesät.

Ergebnis:

In der Kultur A wurden 2 Tage nach DNS-Übertragung mikroskopisch Lymphozyten-Kolonien beobachtet, die in den folgenden 10 Tagen an Größe zunahmen. In der folgenden Woche zerfielen die Kolonien in mehrere kleine Kolonien. Ab der 3. Woche nach DNS-Übertragung wurden in allen Vertiefungen der Kultur A kontinuierlich wachsende Kolonien beobachtet, die zu Zell-Linien auswuchsen (Tab. 2). Ab Tag 50 wurden die Zellen in größere Kulturgefäße überführt und weiter vermehrt. Auch nach 10 Monaten kontinuierlicher Vermehrung in Kultur konnte keine Alterung der Zellen festgestellt werden. In Kultur B wurden nach Schein-DNS-Übertragung keine wachsenden lymphoiden Kolonien beobachtet. Die Zellen alterten und starben innerhalb von 5 Tagen nach Schein-DNS-Übertragung ab.
Die immortalisierten Zellen wurden auf die Ausprägung von Oberflächenmarkern, die charakteristisch für B-Lymphozyten sind, geprüft. Dieses erfolgte mit Hilfe der Schaferythrozytenrosettierung (Kaplan, M.E. et al.: J. Immunol. Methods 5: 131, 1974), der Immunfluoreszenz unter Verwendung von Maus monoklonalen anti-human-pan B- bzw. pan T-Lymphozyten Antikörper (Miles) als primären und Fluoresceinisothiocyanat konjugiertem anti-Maus-IgGl (Miles) als sekundären Antikörper sowie unter Verwendung von fluoreszenzmarkiertem anti-human-Ig Antikörper (Dako) zum Nachweis von B-Lymphozyten Membran-Immunglobulin. Diese Verfahren gehören zu den Standardmethoden der Immunologie. Tabelle 3 zeigt, daß über 90 % der immortalisierten Lymphozyten B-Lymphozyten Marker trugen.

Tabelle 2

| | eingesetzte Lymphozyten | immortalisierte lymphoide Kolonien | eingesetzte DNS ($\mu$g) | immortalisierte Kolonien/$10^7$ Zellen/ $\mu$g DNS |
|---|---|---|---|---|
| Kultur A | $3 \times 10^7$ | 17 | 1 | 5,6 |
| Kultur B | $3 \times 10^7$ | 0 | 0 | 0 |

Tabelle 3

Charakterisierung der immortalisierten Zellen

| Methode | positive Zellen |
|---|---|
| Schaferythrozytenrosettierung | 3 % |
| MAK anti-human-pan B-Lympho- zyten | 96 % |
| MAK anti-human-pan T-Lympho- zyten | 4 % |
| sIg | 91 % |

**Beispiel 3**

Immortalisierung menschlicher T-Lymphozyten aus dem peripheren Blut

Methoden:

Die Präparation menschlicher Lymphozyten aus peripherem Blut erfolgte wie in Beispiel 1 beschrieben. Menschliche T-Lymphozyten wurden mit Hilfe der Affinitäts-Chromatographie aus der Gesamtpopulation der Lymphozyten analog zu dem Verfahren in Beispiel 2 isoliert. Im Unterschied zu dem Verfahren in Beispiel 2 wurde monoklonaler Maus anti-human-pan T-Lymphozyten Antikörper an Sepharose gebunden. Auf diese Weise wurden die T-Lymphozyten an Sepharose gebunden und konnten durch PBS, 0,2 % Humanserumalbumin, 10 mg/ml IgG ausgewaschen werden. Die Reinheit der T-Lymphozyten-Präparation betrug mehr als 90 %, gemessen mit Hilfe der indirekten Immunfluoreszenz des Maus anti-human-pan T-Lymphozyten Antikörpers.

Die T-Lymphozyten wurden durch Inkubation mit "pokeweed mitogen" (PWM, 10 $\mu$g/ml) zum Wachstum angeregt. Nach 2 Tagen wurde die Kultur in zwei Parallelkulturen (A,B) zu je $4 \times 10^7$ Zellen getrennt. In Zellen der Kultur A wurde DNS aus L929-Cytoplasten (1 $\mu$g) übertragen, wie in Beispiel 1 beschrieben. Mit den Zellen der Kultur B wurde eine Schein-DNS-Übertragung durchgeführt. Die Zellen wurden in einer Dichte von $5 \times 10^6$ Zellen/ml in 24-Loch Microtiterplatten ausgesät (1 ml/Vertiefung).

Ergebnis

In Kultur A wurden mehrere wachsende Lymphozytenkolonien beobachtet, die ab Tag 10 nach DNS-Übertragung in kleinere Kolonien zerfielen. In der 3. Woche nach DNS-Übertragung wuchsen kontinuierlich sich vermehrende Lymphozytenkolonien aus. Die Ergebnisse zeigt Tabelle 4. Die Zellen wurden bisher bis zu 6 Monate in Kultur vermehrt, ohne daß eine Alterung der Zellen beobachtet wurde.
In der Kultur B wurde nach Schein-DNS-Übertragung kein Wachstum der Lymphozyten beobachtet. Die Zellen starben innerhalb der nächsten 5 Tage ab.
Die Zellen der Kultur A wurden wie in Beispiel 2 auf ihre Oberflächenmarker untersucht. Über 90 % der Zellen der Kultur A prägten Oberflächenmarker aus, die für T-Lymphozyten charakteristisch sind (siehe Tab. 5).

Tabelle 4

|  | eingesetzte Lymphozyten | immortalisierte lymphoide Kolonien | eingesetzte DNS ($\mu$g) | immortalisierte Kolonien/$10^7$ Zellen/ $\mu$g DNS |
|---|---|---|---|---|
| Kultur A | $4 \times 10^7$ | 5 | 1 | 1,2 |
| Kultur B | $4 \times 10^7$ | 0 | 0 | 0 |

Tabelle 5

Charakterisierung der immortalisierten Zellen

| Methode | Positive Zellen |
|---|---|
| Schaferythrozytenrosettierung | 92 % |
| MAK anti-human-pan B-Lympho-zyten | 4 % |
| MAK anti-human-pan T-Lympho-zyten | 95 % |
| sIg | 5 % |

**Beispiel 4**

Immortalisierung menschlicher Lymphozyten durch Übertragung von DNS aus erfindungsgemäß immortalisierten Lymphozyten.

Methoden:

Menschliche Lymphozyten aus dem peripheren Blut wurden wie in Beispiel 1 beschrieben isoliert.

Die immortalisierende DNS wurde aus Zellen einer erfindungsgemäß (wie im Beispiel 1) immortalisierten lymphoiden Zell-Linie (C-9) gewonnen. Die lymphoide Linie C-9 war durch Übertragung von DNS aus L929-Cytoplasten in Lymphozyten des peripheren Bluts erhalten worden. Die C-9 Zellen wurden in PBS gewaschen und in 200 mM Tris, 100 mM EDTA, 0,2 % SDS, pH 7,2 lysiert. Die RNS wurde durch Inkubation mit RNase A (100 $\mu$g/ml, Boehringer Mannheim) für 2 h bei 37°C, die Proteine durch Inkubation mit Proteinase K (100 $\mu$g/ml, Boehringer Mannheim) für 3 h bei 37°C abgebaut. Die DNS wurde durch Phenol-und Chloroform/Isoamylalkohol (24:1) -Extraktion isoliert.

Die Immortalisierung menschlicher Lymphozyten erfolgte erfindungsgemäß durch Übertragung von DNS aus C-9 Zellen. Dazu wurden die Lymphozyten durch Inkubation mit "pokeweed mitogen" (PWM, 10 $\mu$g/ml) zum Wachstum angeregt. Nach 3 Tagen wurden 2 Parallelkulturen zu je 5 x $10^7$ Lymphozyten angelegt (Kultur A,B). In Zellen der Kultur A wurde (wie in Beispiel 1 beschrieben) DNS aus C-9 Zellen übertragen (10 $\mu$g/$10^7$ Zellen). In Zellen der Kultur B erfolgte eine Schein-DNS-Übertragung wie oben beschrieben.

Ergebnis:

In der Kultur A wuchsen 25 Tage nach DNS-Übertragung Kolonien lymphoider Zellen aus (siehe Tab. 6), die am Tag 56 in größere Kulturgefäße überführt wurden. Die Zellen wurden für zur Zeit 6 Monate ohne erkennbare Zellalterung in Kultur gehalten. Die Zellen in Kultur B zeigten ab Tag 5 nach Schein-DNS-Übertragung Zellalterung und starben innerhalb der folgenden 3 Tage ab.

Ferner wurde mit DNS aus den erfindungsgemäß in Kultur A immortalisierten Zellen (C-17, sekundär transfizierte Zell-Linie) nach Übertragung in Lymphzyten des peripheren Bluts wiederum immortalisierte lymphoide Zell-Linien erzeugt (C-28, tertiär transformierte Zell-Linie). Dabei wurde eine höhere Ausbeute permanent sich teilender lymphoider Kolonien erreicht (siehe Tabelle 7).

Diese Ergebnisse zeigen, daß mit DNS aus erfindungsgemäß immortalisierten Lymphozyten nach Übertragung in menschliche Lymphozyten wiederum permanent wachsende lymphoide Zell-Linien erhalten werden. Dabei wird eine höhere Immortalisierungsfrequenz beobachtet als nach Übertragung von DNS aus L929-Cytoplasten.

Tabelle 6

Immortalisierung menschlicher Lymphozyten durch sekundäre Transfektion

|  | eingesetzte Lymphozyten | immortalisierte lymphoide Kolonien | eingesetzte DNS ($\mu$g) | immortalisierte Kolonien/$10^7$ Zellen/ $\mu$g DNS |
|---|---|---|---|---|
| Kultur A | $5 \times 10^7$ | $\approx 300$ | 50 | 1,2 |
| Kultur B | $5 \times 10^7$ | 0 | 0 | 0 |

Tabelle 7

Immortalisierung menschlicher Lymphozyten durch tertiäre Transfektion

|  | eingesetzte Lymphozyten | immortalisierte lymphoide Kolonien | eingesetzte DNS ($\mu$g) | immortalisierte Kolonien/$10^7$ Zellen/ $\mu$g DNS |
|---|---|---|---|---|
| Kultur A | $5 \times 10^7$ | $\approx 800$ | 50 | 3,2 |
| Kultur B | $5 \times 10^7$ | 0 | 0 | 0 |

**Beispiel 5**

Immortalisierung menschlicher Amnionfibroblasten.

Methoden:

Zur Präparation menschlicher embryonaler Fibroblasten wurde Fruchtwasser durch sterile Punktion der Amnionhöhle Schwangerer gewonnen. Die Zellen wurden durch Zentrifugation bei 100 x g, 10 min sedimentiert, in Iscove's DMEM, 15% fötales Kälberserum, 10 $\mu$g/ml Transferrin, 0,1 U/ml Insulin, 15 mM HEPES, resuspendiert und bei 37°C, 5 % $CO_2$ inkubiert. Nach 10 Tagen wurden die Zellkolonien mit mehr als 30 Zellen/Kolonie subkultiviert.

Die menschlichen Amnionfibroblasten wurden in der 3. Passage in 2 Parallelkulturen (A,B) mit je $10^6$ Zellen geteilt. Die immortalisierende DNS wurde nach dem Verfahren in Beispiel 1 aus L929-Cytoplasten gewonnen. Die Zellen der Kultur A wurden zweimal in HeBS gewaschen und 15 min mit DEAE-Dextran (MW 500.000, 0,1 mg/ml HeBS) bei 37°C inkubiert. Die DNS (0,8 $\mu$g) in 1.200 $\mu$l CaCl (125 mM) wurde tropfenweise zu 1.200 $\mu$l doppelt konzentriertem HeBS, pH 7,1 pipettiert und 20 min bei Raumtemperatur inkubiert. Die Amnionfibroblasten wurden zweimal in HeBS gewaschen und 2 h bei 37°C mit der DNS-Lösung inkubiert. Die Zellen wurden zweimal in HeBS gewaschen und in DMEM, 10 $\mu$g/ml Transferrin, 0,1 U/ml Insulin, 15 mM HEPES, 15% fötalem Kälberserum kultiviert. Die Zellen der Kultur B wurden für eine Schein-DNS-Übertragung verwendet wie oben beschrieben.

Ergebnis:

Die Zellen der Kultur A zeigen ein kontinuierliches Wachstum auch 9 Monate nach DNS-Übertragung, ohne daß eine Zellalterung beobachtet wurde. Die Zellen der Kontrollkultur B alterten nach 50 - 60 Tagen in Kultur und starben ab.

**Beispiel 6**

Übertragung des bakteriellen Gens für Xanthin-guanin-phosphotransferase in immortalisierte menschliche Lymphozyten.

Methoden:

Menschliche Lymphozyten wurden erfindungsgemäß wie in Beispiel 1 beschrieben immortalisiert und zwei Parallelkulturen (A,B) zu je 3 x 10$^7$ Zellen angelegt. In Zellen der Kultur A wurde das Plasmid pSV2gpt (10 $\mu$g) (Mulligan, R.C., Berg, P., Science 209: 1422-1427, 1980), das das bakterielle Gen für die Xanthin-guanin-phosphotransferase (gpt) trägt, übertragen. Bei Zellen der Kultur B erfolgte eine Schein-DNS-Übertragung. Einen Tag nach DNS-Übertragung wurden die Zellen im Selektionsmedium (DMEM, 2 $\mu$g/ml Aminopterin, 250 $\mu$g/ml Xanthin, 15 $\mu$g/ml Hypoxanthin, 150 $\mu$g/ml Glutamin, 10 $\mu$g/ml Thymidin, 25 $\mu$g/ml Mykophenolsäure) kultiviert, um für die Zellen zu selektionieren, die das gpt-Gen aufgenommen haben und das entsprechende Enzym enthalten. Die Zellen, die das gpt-Gen nicht ausprägen, sterben im Selektionsmedium ab. Nach 10 Tagen wurden die überlebenden, im Selektionsmedium sich vermehrenden Kolonien gezählt.

Ergebnis:

Die Kultur A enthielt 10 Tage nach DNS-Übertragung lymphoide Kolonien, die im Selektionsmedium sich vermehren (Tabelle 8). Das gpt-Gen muß in Zellen dieser Kolonien vorhanden sein, da es durch Transfektion mittels DNS aus diesen primären Transfektanten erneut übertragen werden konnte. Im Genom der dabei erhaltenen sekundären Transfektanten wurde das gpt-Gen durch Southern blot Analyse nachgewiesen.

Die Zellen in Kultur B starben nach der Schein-DNS-Übertragung ab, ohne daß wachsende Kolonien im Selektionsmedium erkennbar waren.

Tabelle 8

Übertragung des gpt-Gens in menschliche immortalisierte Lymphozyten

|  | eingesetzte Lymphozyten | Mykophenolsäure resistente Kolonien | eingesetzte DNS (µg) | Mykophenolsäure resistente Kolonien/ µg DNS |
|---|---|---|---|---|
| Kultur A | $3 \times 10^7$ | 156 | 10 | 15,6 |
| Kultur B | $3 \times 10^7$ | 0 | 0 | 0 |

**Beispiel 7**

Immortalisierung menschlicher Lymphozyten durch Übertragung von DNS aus einem Hirt-Überstand von Maus L-Zellen (L-929).

Methoden:

Menschliche Lymphozyten aus dem peripheren Blut wurden wie in Beispiel 1 beschrieben isoliert.

Die immortalisierende DNS wurde aus einem Hirt-Überstand aus Maus L-Zellen (L-929) gewonnen. Die Gewinnung des Hirt-Überstandes erfolgte durch Lyse in Gegenwart von NaCl und SDS nach J. Mol. Biol. 26 (1967) 365-369.

Der Überstand wird mit Proteinase K (100 µg/ml, Boehringer Mannheim) versetzt, 2 Stunden bei 37°C inkubiert und mit Phenol zweimal extrahiert. Anschließend wird der Überstand mit RNase A (100 µg/ml, Boehringer Mannheim) eine Stunde bei 37°C inkubiert, Proteinase K (100 1g/ml, Boehringer Mannheim) hinzugegeben und nochmals 2 Stunden inkubiert. Die DNS wird jeweils zweimal mit Phenol und Chloroform/Isoamylalkohol (24:1) und einmal mit Ether extrahiert, in Ethanol gefällt und in 10 mM/l Tris, 0,1 mM/l EDTA, Ph 7,5, aufgenommen.

Die Übertragung der DNS in menschliche Lymphozyten erfolgte wie im Beispiel 1.3 beschrieben.

Ergebnis:

In Kultur A wurde 3 µg DNS aus dem Hirt-Überstand in $10^8$ Lymphozyten transfiziert. In Kultur B ($10^8$ Lymphozyten) erfolgte eine Schein-DNS-Übertragung. 10 Wochen nach DNS-Übertragung wurden in Kultur A 30 permanent wachsende Kolonien beobachtet. In Kultur B alterten die Zellen innerhalb von 4 Tagen nach Schein-DNS-Übertragung (Tabelle 9).

## T a b e l l e   9

### Immortalisierung menschlicher Lymphozyten durch Übertragung von DNS aus Hirt-Überstand.

|  | einge-setzte Lympho-zyten | immortali-sierte lymphoide Kolonien | einge-setzte DNS (μg) | immortali-sierte Kolonien/$10^7$ Zellen/μg/DNS |
|---|---|---|---|---|
| Kultur A | $10^8$ | 30 | 3 | 1,0 |
| Kultur B | $10^8$ | 0 | 0 | 0 |

**Beispiel 8**

Immortalisierung menschlicher Lymphozyten durch Übertragung von DNS aus einer mitochondreinfreien Cytoplasmafraktion von Maus L-Zellen (L929)

Methoden:

Menschliche Lymphozyten aus dem peripheren Blut werden wie in Beispiel 1 beschrieben, isoliert.

Cytoplasten werden aus L929-Zellen wie in Beispiel 1.2 beschrieben durch Inkubation mit Cytochalasin B gewonnen. Zur Isolierung der Mitochondrien werden die Cytoplasten nach J. Biol. Chem. 249, (1974) 7991-7995 in 134 mM NaCl, 5 mM KCl, 0,7 mM $Na_2HPO_4$, 2,5 mM Tris, pH 7,5, bei 4°C gewaschen und 10 Minuten in 10 mM NaCl, 1,5 mM $CaCl_2$, 10 mM Tris, pH 7,5, bei 4°C inkubiert.

Die Cytoplasten werden durch Homogenisieren lysiert und das 0,7fache Volumen 0,5 M Mannitol, 0,17 M Sucrose, 7 mM Tris, 7 mM EDTA hinzugegeben. Nichtlysierte Cytoplasten werden durch Zentrifugation bei 1.200 x g, 15 Minuten, 4°C sedimentiert. Der mitochondienhaltige Überstand wird in einem diskontinuierlichen Sucrosegradienten (1,0 - 1,5 M Sucrose) in 5 mM EDTA, 10 mM Tris, pH 7,5, bei 65.000 x g, 30 Minuten abgetrennt. Die Mitochondrien reichern sich in der Interphase an, werden gesammelt und in 134 mM NaCl, 5 mM KCl, 0,7 mM $Na_2HPO_4$, 2,5 mM Tris, pH 7,5, durch Zentrifugation bei 20.000 x g, 30 Minuten, 4°C, sedimentiert (mitochondrienreiche Fraktion). Die übrigen Fraktionen des Sucrosegradienten werden vereint (mitochondienarme Fraktion). Das Sediment der itochondienreichen Fraktion wird in 75 mM NaCl, 50 mM EDTA, 25 mM Tris, pH 7,4, 2 % Natriumdodecylsulfat (SDS) resuspendiert. Die DNS wird zweimal in Phenol extrahiert und gegen 150 mM NaCl, 50 mM Nacitrat, pH 7,0, dialysiert.

Das zirkuläre mitochondriale Genom wird in einem CsCl-Ethidiumbromid Gradienten bei 150.000 x g, 24 Stunden, von nicht-mitochondrialer DNS getrennt. Die Bande zirkulärer mitochondrialer DNS wird gewonnen, die DNA dreimal in isopropanol extrahiert und gegen 0,15 M Nacitrat, 0,001 M EDTA, pH 7,2, dialysiert (Fraktion A: zirkulares mitochondriales Genom). Die DNS wird in Ethanol gefällt und in 10 mM Tris, 0,1 mM EDTA, pH 7,5, aufgenommen.

Die mitochondrienarme Fraktion des Sucrose-Gradienten wird zwei Stunden mit Proteinase K (100 μg/ml) inkubiert, zweimal mit Phenol extrahiert, zwei Stunden mit RNase A (100 μg/ml) inkubiert und anschließend zweimal mit Phenol und Chloroform/Isoamylalkohol und einmal mit Ether extrahiert. Die DNS wird in Ethanol gefällt und in 10 mM Tris, 0,1 mM EDTA aufgenommen (Fraktion B: extramitochondriale DNS).

Die Übertragung der DNS der Fraktion A (3 μg) und der Fraktion B (0,2 μg) in je 108 menschliche Lymphozyten erfolgt wie im Beispiel 1.3 beschrieben.

Ergebnis:

In Kultur A werden 3 μg DNS der Fraktion A in $10^8$ Lymphozyten transfiziert. In Kultur B werden 0,2 μg DNS der Fraktion B in $10^8$ Lymphozyten transfiziert. In Kultur C ($10^8$ Lymphozyten) erfolgt eine Schein-DNS-Übertragung.

10 Wochen nach DNS-Übertragung wurden in Kultur B 29 permanent wachsende lymphoide Zellkolonien beobachtet. In Kultur A und C wurden keine permanent wachsende Kolonien erhalten (Tabelle 10).

## T a b e l l e   10

## Immortalisierung menschlicher Lymphozyten durch Transfektion von extramitochondrialer DNS

|  | einge- setzte Lympho- zyten | immortali- sierte lymphoide Kolonien | einge- setzte DNS (μg) | immortali- sierte Kolonien/$10^7$ Zellen/μg/DNS |
|---|---|---|---|---|
| Kultur A | $10^8$ | 0 | 3 | 0 |
| Kultur B | $10^8$ | 29 | 0,2 | 14,5 |
| Kultur C | $10^8$ | 0 | 0 | 0 |

**Patentansprüche**

**1.** Verfahren zur Immortalisierung von humanen oder tierischen Zellen durch Fusion mit einem nicht vermehrungsfähigen Fragment einer permanent züchtbaren Zelle und Züchtung der fusionierten Zellen in einem Kulturmedium ohne Selektionssubstanzen,
**dadurch gekennzeichnet,**
daß man aus dem Cytoplasma einer permanent züchtbaren menschlichen oder tierischen Zelle gewonnene DNS in die zu immortalisierenden Zellen transfiziert.

**2.** Verfahren nach Anspruch 1,
**dadurch gekennzeichnet,**
daß man als permanent züchtbare Zelle eine Tumorzelle oder eine immortalisierte, nicht tumorigene Zelle verwendet.

**3.** Verfahren nach Anspruch 1 oder 2,
**dadurch gekennzeichnet,**
daß man zur Gewinnung der DNS Cytoplasten der permanent züchtbaren Zelle lysiert, das lösliche Lysat mit einer Proteinase inkubiert, dann die DNS abtrennt und mit RNase inkubiert und erneut mit einem DNS-Lösungsmittel extrahiert.

**4.** Verfahren nach Anspruch 1 oder 2,
**dadurch gekennzeichnet,**
daß man zur Gewinnung der DNS Cytoplasma der permanent züchtbaren Zelle lysiert, die nukleäre DNS abtrennt, den Überstand mit einer Proteinase inkubiert, dann die DNS abrennt und mit RNase inkubiert und erneut mit einem DNS-Lösungsmittel extrahiert.

**5.** Verfahren nach Anspruch 1 oder 2,
**dadurch gekennzeichnet,**
daß man zur Gewinnung von DNS Cytoplasma der permanent züchtbaren Zelle lysiert, die mitochondrienfreie Fraktion gewinnt, das lösliche Lysat mit einer Proteinase inkubiert, dann die DNS abtrennt und mit RNase inkubiert und erneut mit einem DNS-Lösungsmittel extrahiert.

**6.** Verfahren nach Anspruch 3, 4 oder 5
**dadurch gekennzeichnet,**
daß man zur Abtrennung mit einem DNS-Lösungsmittel wie Phenol und/oder Chloroform-Isoamylalkoholgemisch extrahiert.

**7.** Verfahren nach einem der vorhergehenden Ansprüche,
**dadurch gekennzeichnet,**
daß man die zu immortalisierende Zelle in zum Wachstum bzw. zur Teilung angeregten Zustand mit der cytoplasmatischen DNS transfiziert.

**8.** Verfahren nach Anspruch 7,
**dadurch gekennzeichnet,**
daß man Lymphozyten in der Proliferationsphase I verwendet.

**Claims**

**1.** Process for the immortalization of human or animal cells by fusion with a fragment of a permanently culturable cell which is not capable of proliferation and culturing the fused cells in a culture medium without selective substances,
**wherein**
DNA obtained from the cytoplasm of a permanently culturable human or animal cell is transfected into the cells to be immortalized.

**2.** Process as claimed in claim 1,
**wherein**
a tumour cell or an immortalized, non-tumorigenic cell is used as the permanently culturable cell.

**3.** Process as claimed in claim 1 or 2,
**wherein**
in order to obtain the DNA, cytoplasts of the permanently culturable cells are lysed, the soluble lysate is incubated with a proteinase, the DNA is then isolated, incubated with RNase and again extracted with a DNA solvent.

**4.** Process as claimed in claim 1 or 2,
**wherein**
in order to obtain the DNA, cytoplasm of the permanently culturable cell is lysed, the nuclear DNA is separated, the supernatant is incubated with a proteinase, the DNA is then isolated and incubated with RNase and again extracted with a DNA solvent.

**5.** Process as claimed in claim 1 or 2,
**wherein**
in order to obtain DNA, cytoplasm of the permanently culturable cell is lysed, the mitochondria-free fraction is isolated, the soluble lysate is incubated with a proteinase, the DNA is then isolated and incubated with RNase and again extracted with a DNA solvent.

**6.** Process as claimed in claim 3, 4 or 5,
**wherein**
for the isolation, an extraction is carried out with a DNA solvent such as phenol and/or a chloroform-isoamyl alcohol mixture.

**7.** Process as claimed in one of the previous claims,
**wherein**
the cell to be immortalized is transfected with the cytoplasmic DNA in a state stimulated to growth or division.

**8.** Process as claimed in claim 7,
**wherein**
lymphocytes in proliferation phase 1 are used.

**Revendications**

**1.** Procédé pour immortaliser des cellules humaines ou animales par fusion avec un fragment incapable de multiplication d'une cellule capable d'être cultivée de manière permanente et culture des cellules fusionnées dans un milieu de culture sans substances de sélection, caractérisé en ce que l'on transfecte dans les cellules à immortaliser l'ADN obtenu à partir du cytoplasme d'une cellule humaine ou animale capable d'être cultivée de manière permanente.

**2.** Procédé selon la revendication 1, caractérisé en ce que l'on utilise comme cellule capable d'être cultivée de manière permanente une cellule tumorale ou une cellule non tumorigène immortalisée.

**3.** Procédé selon la revendication 1 ou 2, caractérisé en ce que, pour obtenir l'ADN, on lyse des organites du cytoplasme de la cellule capable d'être cultivée de manière permanente, on incube le lysat soluble avec une protéinase puis on sépare l'ADN, on l'incube avec la ribonucléase et on extrait de nouveau avec un solvant de l'ADN.

**4.** Procédé selon la revendication 1 ou 2, caractérisé en ce que, pour obtenir l'ADN, on lyse le cytoplasme de la cellule capable d'être cultivée de manière permanente, on sépare l'ADN nucléaire, on incube le surnageant avec une protéinase puis on sépare l'ADN, on l'incube avec la ribonucléase et on extrait de nouveau avec un solvant de l'ADN.

**5.** Procédé selon la revendication 1 ou 2, caractérisé en ce que, pour obtenir l'ADN, on lyse le cytoplasme de la cellule capable d'être cultivée de manière permanente, on recueille la fraction exempte de mitochondries, on incube le lysat soluble avec une protéinase puis on sépare l'ADN, on l'incube avec la ribonucléase et on extrait de nouveau avec un solvant de l'ADN.

**6.** Procédé selon la revendication 3, 4 ou 5, caractérisé en ce que, pour la séparation, on extrait avec un solvant de l'ADN comme le phénol et/ou un mélange chloroforme-alcool isoamylique.

**7.** Procédé selon l'une des revendications précédentes, caractérisé en ce que l'on transfecte avec l'ADN cytoplasmique la cellule à immortaliser à l'état stimulé pour la croissance ou la division.

**8.** Procédé selon la revendication 7, caractérisé en ce que l'on utilise des lymphocytes dans la phase de prolifération I.